# EUROPÄISCHE PATENTSCHRIFT

(11) **EP 1 265 699 B1**
(45) Veröffentlichungstag und Bekanntmachung des Hinweises auf die Patenterteilung: **29.06.2005**
(21) Anmeldenummer: 01907573.8
(22) Anmeldetag: 01.03.2001
(51) Int. Cl.: B01J 19/00, C07C 6/02

(54) **VERFAHREN ZUR METATHESEREAKTION UNGESÄTTIGTER ORGANISCHER VERBINDUNGEN**
METHOD FOR CARRYING OUT A METATHESIS REACTION OF UNSATURATED ORGANIC COMPOUNDS
PROCEDE POUR REALISER LA METATHESE DE COMPOSES ORGANIQUES INSATURES

(30) Priorität: 23.03.2000 DE 10014297
(43) Veröffentlichungstag der Anmeldung: 18.12.2002
(73) Patentinhaber: MERCK PATENT GmbH, 64293 Darmstadt (DE)
(72) Erfinder: WURZIGER, Hanns, 64291 Darmstadt (DE); PIEPER, Guido, 64291 Darmstadt (DE); SCHWESINGER, Norbert, 98693 Ilmenau (DE)
(86) Internationale Anmeldenummer: PCT/EP2001/002301
(87) Internationale Veröffentlichungsnummer: WO 2001/070387

(56) Entgegenhaltungen:
- EP-A- 0 456 373
- WO-A-00/51720
- WO-A-99/51344
- DE-A- 4 433 439

## Beschreibung

Die vorliegende Erfindung betrifft ein Verfahren zur Metathesereaktion ungesättigter organischer Verbindungen.

Die Metathesereaktion geeigneter ungesättigter organischer Verbindungen ist ein in der chemischen Industrie sehr häufig durchgeführtes Verfahren, dessen große Bedeutung sich auch in zahlreichen Veröffentlichungen zu diesem Thema widerspiegelt.

Die Durchführung von Metathesereaktionen im technischen Maßstab bringt jedoch Sicherheitsprobleme und Gefahren mit sich. Zum einen werden häufig größere Mengen hochgiftige chemische Substanzen eingesetzt, die für sich allein bereits ein erhebliches Risiko für Mensch und Umwelt darstellen und zum anderen können in vielen Fällen die Reaktionsbedingungen nur mit beträchtlichem Aufwand gut kontrolliert werden. Ferner ist bei solchen Metathesereaktionen im technischen Maßstab die Verwirklichung und Aufrechterhaltung von Schutzgasbedingungen oft sehr aufwendig.

Aufgabe der vorliegenden Erfindung ist es daher, ein Verfahren zur Metathesereaktion ungesättigter organischer Verbindungen zur Verfügung zu stellen, das die oben genannten Nachteile vermeidet. Dieses Verfahren soll insbesondere in einfacher, reproduzierbarer Weise mit erhöhter Sicherheit für Mensch und Umwelt sowie mit guten Ausbeuten durchführbar sein, die Reaktionsbedingungen sollen sehr gut kontrollierbar sein und die zur Durchführung der Reaktion notwendigen Schutzgasbedingungen sollen ohne großen technischen Aufwand realisiert werden können.

Die Lösung dieser Aufgabe gelingt überraschenderweise durch das erfindungsgemäße Verfahren zur Metathesereaktion ungesättigter organischer Verbindungen, bei dem wenigstens eine ungesättigte organische Verbindung in flüssiger oder gelöster Form mit wenigstens einem Metathesekatalysator in flüssiger oder gelöster Form in wenigstens einem Mikroreaktor vermischt wird, während einer Verweilzeit reagiert und die gebildete organische Verbindung gegebenenfalls aus dem Reaktionsgemisch isoliert wird.

Vorteilhafte Ausführungsformen des erfindungsgemäßen Verfahrens sind in den Unteransprüchen beschrieben.

Erfindungsgemäß können einzelne ungesättigte organische Verbindungen oder Gemische aus mindestens zwei dieser Verbindungen nach dem beanspruchten Verfahren umgesetzt werden. Vorzugsweise werden einzelne ungesättigte organische Verbindungen bei dem erfindungsgemäßen Verfahren eingesetzt.

In einer besonders bevorzugten Ausführungsform handelt es sich bei der Metathesereaktion um eine ringschließende Metathesereaktion einer einzelnen ungesättigten organischen Verbindung.

Ein Mikroreaktor im Sinne der Erfindung ist ein Reaktor mit einem Volumen ≤ 1000 µl in dem die Flüssigkeiten und/oder Lösungen wenigstens einmal innig vermischt werden. Vorzugsweise beträgt das Volumen des Reaktors ≤ 100 µl, besonders bevorzugt ≤ 50 µl.

Der Mikroreaktor wird bevorzugt aus dünnen, miteinander verbundenen Siliziumstrukturen hergestellt.

Vorzugsweise ist der Mikroreaktor ein miniaturisierter Durchflußreaktor, besonders bevorzugt ein statischer Mikromischer. Ganz besonders bevorzugt ist der Mikroreaktor ein statischer Mikromischer, wie er in der Patentanmeldung mit der internationalen Veröffentlichungsnummer WO 96/30113 beschrieben ist, die hiermit als Referenz eingeführt wird und als Teil der Offenbarung gilt. Ein solcher Mikroreaktor weist kleine Kanäle auf, in denen Flüssigkeiten und/oder in Lösungen vorliegende, chemische Verbindungen durch die kinetische Energie der strömenden Flüssigkeiten und/oder Lösungen miteinander vermischt werden.

Gemäß WO 96/30113 weist ein solcher Mikroreaktor mindestens ein Mischelement auf mit mindestens einem Einlasskanal und mindestens einem Auslasskanal, wobei
- von dem Einlasskanal mindestens zwei Mikrokanäle ausgehen und alle abgehenden Kanäle in einer Verzweigungsebene liegen,
- die Mikrokanäle einem Zusammenflusselement zugeführt werden, wobei der Zufluß in einer Ebene erfolgt, die gegenüber der Verzweigungsebene um 90° verdreht angeordnet ist und
- das Mischelement in der planaren Oberfläche eines Substrates angeordnet ist, wobei die planare Oberfläche des Substrates mit einer Abdeckung hermetisch dicht verschlossen ist.
Die Grundanordnung eines entsprechenden Mischelements wird in WO 96/30113 schematisch durch Fig. 1a und Fig. 1b wiedergegeben. In einem Mischelement gemäß Fig. 1a wird eine Grundanordnung eines Mischelements für vertikale Aufteilung und horizontale Übereinanderschichtung der Flüssigkeiten gezeigt. Hierin wird die Flüssigkeit durch einen Mikrokanal am Eingang 1 in das Mischelement eingeleitet. Dieser Mikrokanal besitzt eine Verzweigung 2, von dem die Mikrokanäle 3 und 4 ausgehen. Die Verzweigung 2 bewirkt eine Aufteilung der Flüssigkeit entlang einer gedachten vertikalen Linie. Der Eingang 1, die Verzweigung 2 und die Mikrokanäle 3 und 4 befinden sich in einer horizontalen Ebene. Die nachfolgenden Mikrokanäle sind so angeordnet, dass Mikrokanal 6 diese Ebene verlässt und Mikrokanal 5 in der horizontalen Ebene bleibt. Die Mikrokanäle sind so angeordnet, dass sie in einem Zusammenflusselement 7 wieder zusammentreffen, wobei sich die beiden Mikrokanäle in uriterschiedlichen Ebenen befinden. Dadurch erfolgt am Zusammenflusselement 7 entlang einer gedachten horizontalen Linie, die Übereinanderschichtung der aus den Mikrokanälen 5 und 6 strömenden Flüssigkeiten. Nachdem die Flüssigkeiten das Zusammenflusselement 7 passiert haben, gelangen sie in einen weiteren Mikrokanal 8. Dieser kann einen erneuten Eingang in ein folgendes Mischelement bilden oder zum Ausgang des Mikromischers führen.
In Fig. 1b wird eine Grundanordnung eines Mischelements für horizontale Aufteilung und vertikale Aneinanderschichtung der Flüssigkeiten gezeigt.
In Fig. 2 der Schrift WO 96/30113 wird die Ausgestaltung eines entsprechenden Mikromischers in Gabelform wiedergegeben. Hiervon können beliebig viele in einem Substrat angeordnet sein.

Die Kanäle des Mikroreaktors weisen vorzugsweise einen Durchmesser von 10 bis 1000 µm, besonders bevorzugt von 20 bis 800 µm und ganz besonders bevorzugt von 30 bis 400 µm auf.

Vorzugsweise werden die Flüssigkeiten und/oder Lösungen so in den Mikroreaktor gepumpt, daß sie diesen mit einer Durchflußgeschwindigkeit von 0,01 µl/min bis 100 ml/min, besonders bevorzugt 1 µl/min bis 1 ml/min durchströmen.

Der Mikroreaktor ist erfindungsgemäß vorzugsweise temperierbar.

Erfindungsgemäß ist der Mikroreaktor vorzugsweise über einen Auslaß mit wenigstens einer Verweilstrecke, vorzugsweise einer Kapillare, besonders bevorzugt einer temperierbaren Kapillare verbunden. In diese Verweilstrecke bzw. Kapillare werden die Flüssigkeiten und/oder Lösungen nach ihrer Durchmischung im Mikroreaktor zur Verlängerung ihrer Verweilzeit geführt.

Die Verweilzeit im Sinne der Erfindung ist die Zeit zwischen der Durchmischung der Edukte und der Aufarbeitung der resultierenden Reaktionslösung zur Analyse bzw. Isolierung der (des) gewünschten Produkte(s).

Die erforderliche Verweilzeit bei dem erfindungsgemäßen Verfahren hängt von verschiedenen Parametern ab, wie z.B. der Temperatur oder der Reaktivität der Edukte. Dem Fachmann ist es möglich, die Verweilzeit an diese Parameter anzupassen und so einen optimalen Reaktionsverlauf zu erzielen.

Die Verweilzeit der Reaktionslösung in dem zum Einsatz kommenden System aus wenigstens einem Mikroreaktor und gegebenenfalls einer Verweilstrecke kann durch die Wahl der Durchflußgeschwindigkeit der eingesetzten Flüssigkeiten und/oder Lösungen eingestellt werden.

Ebenfalls bevorzugt wird das Reaktionsgemisch durch zwei oder mehr in Reihe geschaltete Mikroreaktoren geführt. Hierdurch wird erreicht, daß auch bei erhöhter Durchflußgeschwindigkeit die Verweilzeit verlängert wird und die eingesetzten Komponenten der Metathesereaktion so umgesetzt werden, daß eine optimale Produktausbeute der gewünschten organischen Verbindung(en) erreicht wird.

In einer weiteren bevorzugten Ausführungsform wird das Reaktionsgemisch durch zwei oder mehr parallel angeordnete Mikroreaktoren geleitet, um den Durchsatz zu erhöhen.

In einer anderen bevorzugten Ausführungsform des erfindungsgemäßen Verfahrens wird die Zahl und die Anordnung der Kanäle in einem öder mehreren Mikroreaktor(en) so variiert, daß die Verweilzeit verlängert wird, so daß auch hier bei erhöhter Durchflußgeschwindigkeit eine optimale Ausbeute an der (den) gewünschten organischen Verbindung(en) erreicht wird.

Vorzugsweise beträgt die Verweilzeit der Reaktionslösung im Mikroreaktor, gegebenfalls im Mikroreaktor und der Verweilstrecke ≤ 15 Stunden, besonders bevorzugt ≤ 3 Stunden, ganz besonders bevorzugt ≤ 1 Stunde.

Das erfindungsgemäße Verfahren kann in einem sehr breiten Temperaturbereich durchgeführt werden, der im wesentlichen durch die Temperaturbeständigkeit der zum Bau des Mikroreaktors, gegebenenfalls der Verweilstrecke, sowie weiterer Bestandteile, wie z.B. Anschlüsse und Dichtungen, eingesetzten Materialien und durch die physikalischen Eigenschaften der eingesetzten Lösungen und/oder Flüssigkeiten beschränkt ist. Vorzugsweise wird das erfindungsgemäße Verfahren bei einer Temperatur von -100 bis +250 °C, besonders bevorzugt von -78 bis +150 °C und ganz besonders bevorzugt von 0 bis +40 °C durchgeführt.

Das erfindungsgemäße Verfahren kann sowohl kontinuierlich als auch diskontinuierlich durchgeführt werden. Vorzugsweise wird es kontinuierlich durchgeführt.

Für die Durchführung des erfindungsgemäßen Verfahrens zur Metathesereaktion ungesättigter organischer Verbindungen ist es erforderlich, daß die Metathesereaktion möglichst in homogener flüssiger Phase, die keine oder nur sehr kleine Feststoffpartikel enthält, durchgeführt wird, da sonst die in den Mikroreaktoren vorhandenen Kanäle verstopft werden.

Der Reaktionsverlauf der Metathesereaktion in dem erfindungsgemäßen Verfahren kann mit verschiedenen dem Fachmann bekannten analytischen Methoden verfolgt und gegebenenfalls geregelt werden. Vorzugsweise wird der Reaktionsverlauf chromatographisch, besonders bevorzugt gaschromatographisch und/oder durch Hochdruckflüssigkeitschromatographie verfolgt und gegebenenfalls geregelt. Die Kontrolle der Reaktion ist bei dem erfindungsgemäßen Verfahren im Vergleich zu bekannten Verfahren deutlich verbessert.

Nach der Reaktion werden die gebildeten organischen Verbindungen gegebenenfalls isoliert. Vorzugsweise wird (werden) das (die) gebildete(n) Produkt(e) durch Extraktion aus dem Reaktionsgemisch isoliert.

Als ungesättigte organische Verbindungen können bei dem erfindungsgemäßen Verfahren alle dem Fachmann als Substrate von Metathesereaktionen bekannten ungesättigten organischen Verbindungen eingesetzt werden. Vorzugsweise werden die ungesättigten organischen Verbindungen aus aliphatischen, aromatischen oder heteroaromatischen Alkenen ausgewählt.

Als aliphatische Alkene können alle dem Fachmann bekannten aliphatischen Alkene eingesetzt werden, die sich als Substrat für Metathesereaktionen eignen. Dabei sind auch geradkettige und verzweigte Alkene umfaßt.

Als aromatische Alkene können alle dem Fachmann bekannten aromatischen Alkene eingesetzt werden, die sich als Substrat für Metathesereaktionen eignen. Im Sinne der Erfindung werden damit Verbindungen und/oder Derivate umfaßt, die ein monocyclisches und/oder polycyclisches homoaromatisches Grundgerüst oder eine entsprechende Teilstruktur, z.B. in Form von Substituenten, aufweisen.

Als heteroaromatische Alkene können alle dem Fachmann bekannten heteroaromatischen Alkene eingesetzt werden, die sich als Substrat für Metathesereaktionen eignen und die wenigstens ein Heteroatom enthalten. Heteroaromatische Verbindungen im Sinne der Erfindung umfassen heteroaromatische Verbindungen und/oder deren Derivate, die wenigstens ein monocyclisches und/oder polycyclisches heteroaromatisches Grundgerüst oder eine entsprechende Teilstruktur, z.B. in Form von Substituenten, aufweisen. Heteroaromatische Grundgerüste oder Teilstrukturen umfassen besonders bevorzugt wenigstens ein Sauerstoff-, Stickstoff- oder Schwefelatom.

Als Metathesekatalysatoren können bei dem erfindungsgemäßen Verfahren sämtliche, dem Fachmann bekannten, für Metathesereaktionen geeigneten Metathesekatalysatoren oder eine Mischung aus mindestens zwei Katalysatoren eingesetzt werden. Vorzugsweise wird jeweils nur ein Metathesekatalysator bei dem erfindungsgemäßen Verfahren verwendet.

In einer weiteren bevorzugten Ausführungsform wird wenigstens ein Metathesekatalysator ausgewählt aus Carben- oder Carbinkomplexen oder ein Gemisch dieser Komplexe eingesetzt.

In einer besonders bevorzugten Ausführungsform wird als Carbenkomplex wenigstens ein Komplex ausgewählt aus Bis-(tricyclohexylphosphin)-benzylidenrutheniumdichlorid (Cl₂(Cy₃P)₂Ru=CHPh, "Grubbs"-Katalysator), einer Variante oder einem Derivat des "Grubbs"-Katalysators, 2,6-Diisopropylphenylimido-neophylidenmolybdän-bis-(hexafluor-tert.-butoxid) (2,6-iPr₂C₆H₃N=Mo{OC(CF₃)₂Me}₂=CHCMe₂Ph, "Schrock"-Katalysator), einer Variante oder einem Derivat des "Schrock"-Katalysators oder ein Gemisch der vorstehend genannten Komplexe verwendet.

Das molare Verhältnis von eingesetzter ungesättigter organischer Verbindung zu eingesetztem Metathesekatalysator hängt bei dem erfindungsgemäßen Verfahren von der Reaktivität der eingesetzten ungesättigten organischen Verbindungen und der Metathesekatalysatoren ab. Vorzugsweise wird die ungesättigte organische Verbindung und der Metathesekatalysator in einem äquimolaren Verhältnis verwendet. In einer anderen bevorzugten Ausführungsform wird die ungesättigte organische Verbindung bis zu einem 10000fachen molaren Überschuß, besonders bevorzugt in einem 10fachen bis 100fachen Überschuß, ganz besonders bevorzugt in einem 20fachen bis 30fachen Überschuß bezogen auf den Metathesekatalysator eingesetzt.

Die Selektivität der Reaktion selbst hängt außer von der Konzentration der eingesetzten Reagenzien von einer Reihe weiterer Parameter, wie z.B. der Temperatur, der Art des verwendeten Metathesekatalysators oder der Verweilzeit, ab. Dem Fachmann ist es möglich, die verschiedenen Parameter auf die jeweilige Metathesereaktion so abzustimmen, daß das (die) gewünschte(n) Produkt(e) erhalten wird (werden).

Für das erfindungsgemäße Verfahren ist es wesentlich, daß die eingesetzten ungesättigten organischen Verbindungen und Metathesekatalysatoren entweder selbst flüssig sind oder in gelöster Form vorliegen. Sofern diese Verbindungen nicht schon selbst in flüssiger Form vorliegen, müssen sie daher vor der Durchführung des erfindungsgemäßen Verfahrens in einem geeigneten Lösungsmittel gelöst werden. Als Lösungsmittel werden bevorzugt Wasser, halogenierte Lösungsmittel, besonders bevorzugt Dichlormethan, Chloroform, 1,2-Dichlorethan oder 1,1,2,2-Tetrachlorethan, geradkettige, verzweigte oder cyclische Paraffine, besonders bevorzugt Pentan, Hexan, Heptan, Octan, Cyclopentan, Cycloheptan oder Cyclooctan oder geradkettige, verzweigte oder cyclische Ether, besonders bevorzugt Diethylether, Methyl-tert.-butylether, Tetrahydrofuran oder Dioxan, aromatische Lösungsmittel, besonders bevorzugt Toluol, Xylole, Ligroin, Phenylether oder Gemische dieser Lösungsmittel eingesetzt.

Bei dem erfindungsgemäßen Verfahren ist die Gefahr für Mensch und Umwelt durch austretende Chemikalien erheblich verringert und führt somit zu einer erhöhten Sicherheit beim Umgang mit Gefahrstoffen. Die Metathesereaktion ungesättigter organischer Verbindungen nach dem erfindungsgemäßen Verfahren ermöglicht ferner eine bessere Kontrolle der Reaktionsbedingungen, wie z.B. Reaktionsdauer und Reaktionstemperatur, als dies in den herkömmlichen Verfahren möglich ist. Die Temperatur kann in jedem Volumenelement des Systems individuell gewählt und konstant gehalten werden. Der Reaktionsverlauf der Metathesereaktion ist bei dem erfindungsgemäßen Verfahren sehr schnell und genau regelbar. Schutzgasbedingungen lassen sich sehr leicht verwirklichen und aufrechterhalten. Die gebildeten organischen Produkte lassen sich so in sehr guten und reproduzierbaren Ausbeuten erhalten.

Besonders vorteilhaft ist auch, daß das erfindungsgemäße Verfahren kontinuierlich durchgeführt werden kann. Hierdurch ist es im Vergleich zu herkömmlichen Verfahren schneller und kostengünstiger und es ist ohne großen Meß- und Regelungsaufwand möglich, beliebige Mengen der gewünschten ungesättigten organischen Verbindungen herzustellen.

Im Folgenden wird die Erfindung anhand eines Beispiels erläutert. Dieses Beispiel dient lediglich der Erläuterung der Erfindung und schränkt den allgemeinen Erfindungsgedanken nicht ein.

### Beispiel

### Ringschließende Metathesereaktion von 1,7-Octadien zu Cyclohexen

Die ringschließende Metathesereaktion von 1,7-Octadien mit Bis-(tricyclohexylphosphin)-benzylidenrutheniumdichlorid ("Grubbs"-Katalysator) erfolgte in einem statischen Mikromischer (Technische Universität Ilmenau, Fakultät Maschinenbau, Dr.-Ing. Norbert Schwesinger, Postfach 100565, D-98684, limenau) mit einer Baugröße von 40 mm x 25 mm x 1 mm, der insgesamt 11 Mischstufen mit einem Volumen von jeweils 0,125 µl aufwies. Der Gesamtdruckverlust betrug circa 1000 Pa.

Der statische Mikromischer war über einen Auslaß und eine Omnifit Mitteldruck-HPLC-Verbindungskomponente (Omnifit, Großbritannien) an eine Teflon-Kapillare mit einem Innendurchmesser von 0,49 mm und einer Länge von 5,0 m verbunden. Die Reaktion wurde bei Raumtemperatur durchgeführt.

Es wurde eine 2 ml Einweginjektionsspritze mit einem Teil einer Lösung aus 110 mg (1 mmol) 1,7-Octadien und 5 ml Dichlormethan und eine weitere 2 ml Spritze mit einem Teil einer Lösung aus 40 mg (0,05 mmol) Bis-(tricyclohexylphosphin)-benzylidenrutheniumdichlorid ("Grubbs"-Katalysator) in 5 ml Dichlormethan befüllt. Anschließend wurde der Inhalt beider Spritzen mit einer Dosierpumpe (Harvard Apparatus Inc., Pump 22, South Natick, Massachussets, USA) in den statischen Mikromischer überführt. Die Versuchsanordnung wurde vor der Durchführung der Reaktion in Bezug auf die Abhängigkeit der Verweilzeit von der Pumpenflußrate kalibriert. Die Pumprate wurde so eingestellt, daß eine Durchflußgeschwindigkeit von 20 µl/min und damit eine Verweilzeit von circa 3,5 Stunden resultieren sollte. Aufgrund der Bildung von gasförmigem Ethen in der Metathesereaktion war die Verweilzeit im Reaktor und damit die Reaktionszeit deutlich geringer und betrug circa 1 Stunde. Die Reaktionen wurden mit Hilfe eines Merck Hitachi LaChrom HPLC-Instruments verfolgt. Am Ende der Reaktion wurde der vollständige Umsatz des eingesetzten 1,7-Octadiens mittels eines Hewlett-Packard GC-MS-Instruments festgestellt. Neben geringen Mengen Toluol, Styrol und Tricyclohexylphosphin war Cyclohexen das einzige gefundene Produkt.

## Patentansprüche

1. Verfahren zur Durchführung von Metathesereaktionen ungesättigter organischer Verbindungen, **dadurch gekennzeichnet, dass** wenigstens eine ungesättigte organische Verbindung, ausgewählt aus den aliphatischen, aromatischen oder heteroaromatischen Alkenen, in flüssiger oder gelöster Form mit wenigstens einem Metathesekatalysator, ausgewählt aus den Carben- oder Carbinkomplexen oder ein Gemisch aus diesen Katalysatoren in flüssiger oder gelöster Form miteinander in wenigstens einem Mikroreaktor vermischt werden und die ungesättigten organischen Verbindungen während einer Verweilzeit von ≤ 1 Stunde im Mikroreaktor oder in einem über einen Auslaß mit einer Kapillare verbundenen Mikroreaktor, reagieren und die gebildeten organischen Verbindungen gegebenenfalls aus dem Reaktionsgemisch isoliert werden, und dass es sich bei dem Mikroreaktor um einen Durchflussreaktor in Form eines statischen Mikromischers mit einem Volumen von ≤ 100 µl und kleinen Kanälen mit einem Durchmesser von 10 bis 1000 µm handelt, der mit einer Durchflussgeschwindigkeit von 0,01 µl/min bis 100 ml/min durchströmt wird, und worin die Flüssigkeiten und/oder Lösungen ausgehend von einem Einlasskanal jeweils in einer vertikalen Ebene in zwei in Mikrokanälen fließende Teilströme geteilt werden, anschließend den abgehenden Mikrokanälen folgend in einem Zusammenflusselement in einer gegenüber der Verzweigungsebene um 90 ° verdreht angeordneten Ebene übereinander geschichtet werden.

2. Verfahren nach Anspruch 1, **dadurch gekennzeichnet, dass** das Volumen des Mikroreaktors ≤ 50 µl beträgt.

3. Verfahren gemäß Anspruch 1, **dadurch gekennzeichnet, dass** ein Mikroreaktor mit 11 Mischstufen mit einem Volumen von jeweils 0,125 µl verwendet wird.

4. Verfahren nach Anspruch 1, **dadurch gekennzeichnet, dass** der Mikroreaktor über einen Auslass mit einer temperierbaren Kapillare verbunden ist.

5. Verfahren nach einem der Ansprüche 1 bis 4, **dadurch gekennzeichnet, dass** der Mikroreaktor temperierbar ist.

6. Verfahren nach einem der Ansprüche 1 bis 5, **dadurch gekennzeichnet, dass** der Mikroreaktor Kanäle mit einem Durchmesser von 20 bis 800 µm, besonders bevorzugt von 30 bis 400 µm, aufweist.

7. Verfahren nach einem der Ansprüche 1 bis 6, **dadurch gekennzeichnet, dass** das Reaktionsgemisch den Mikroreaktor mit einer Durchflussgeschwindigkeit von 1 µl/min bis 1 ml/min durchströmt.

8. Verfahren nach einem der Ansprüche 1 bis 7, **dadurch gekennzeichnet, dass** es bei einer Temperatur von -100 bis +250 °C, vorzugsweise von - 78 bis +150 °C, besonders bevorzugt von 0 bis +40 °C durchgeführt wird.

9. Verfahren nach einem der Ansprüche 1 bis 8, **dadurch gekennzeichnet, dass** die Metathesereaktion eine ringschließende Metathesereaktion einer ungesättigten organischen Verbindung ist und als Katalysator ein Carbenkomplex ausgewählt aus Bis-(tricyclohexylphosphin)-benzylidenrutheniumdichlorid (Cl₂(Cy₃P)₂Ru=CHPh, "Grubbs"-Katalysator), einer Variante oder einem Derivat des "Grubbs"-Katalysators, 2,6-Diisopropylphenylimidoneophylidenmolybdän-bis-(hexafluor-tert.-butoxid) (2,6-iPr₂C₆H₃N=Mo{OC(CF₃)₂Me}₂=CHCMe₂Ph, "Schrock"-Katalysator),einer Variante oder eines Derivats des "Schrock"-Katalysators oder ein Gemisch der vorstehend genannten Komplexe verwendet wird, wobei die ungesättigte organische Verbindung in einem äquimolaren Verhältnis, oder bis zu einem 10000fachen molaren Überschuss, besonders bevorzugt in einem 10fachen bis 100fachen Überschuss, ganz besonders bevorzugt in einem 20fachen bis 30fachen Überschuss bezogen auf den Metathesekatalysator eingesetzt wird.

10. Verfahren nach einem der Ansprüche 1 bis 9, **dadurch gekennzeichnet, dass** der Reaktionsverlauf chromatographisch, vorzugsweise gaschromatographisch und/oder durch Hochdruckflüssigkeitschromatographie verfolgt und gegebenenfalls geregelt wird.

## Claims

1. Process for carrying out metathesis reactions of unsaturated organic compounds, **characterised in that** at least one unsaturated organic compound, selected from the aliphatic, aromatic or heteroaromatic alkenes, in liquid or dissolved form is mixed with at least one metathesis catalyst, selected from the carbene or carbyne complexes or a mixture of these catalysts in liquid or dissolved form in at least one microreactor, and the unsaturated organic compounds react for a residence time of ≤ 1 hour in the microreactor or in a microreactor connected to a capillary via an outlet, and the organic compounds formed are optionally isolated from the reaction mixture, and **in that** the microreactor is a flow reactor in the form of a static micromixer having a volume of ≤ 100 µl and small channels having a diameter of 10 to 1000 µm through which flow takes place at a flow rate of 0.01 µl/min to 100 ml/min, and in which the liquids and/or solutions are split, starting from an inlet channel, in each case in a vertical plane, into two substreams flowing in microchannels, the emanating microchannels are subsequently layered one on top of the other in a confluence element in a plane arranged rotated by 90° compared with the branching plane.

2. Process according to Claim 1, **characterised in that** the volume of the microreactor is ≤ 50 µl.

3. Process according to Claim 1, **characterised in that** a microreactor having 11 mixing stages each with a volume of 0.125 µl is used.

4. Process according to Claim 1, **characterised in that** the microreactor is connected to a heatable capillary via an outlet.

5. Process according to one of Claims 1 to 4, **characterised in that** the microreactor is heatable.

6. Process according to one of Claims 1 to 5, **characterised in that** the microreactor has channels having a diameter of 20 to 800 µm, particularly preferably of 30 to 400 µm.

7. Process according to one of Claims 1 to 6, **characterised in that** the reaction mixture flows through the microreactor at a flow rate of 1 µl/min to 1 ml/min.

8. Process according to one of Claims 1 to 7, **characterised in that** it is carried out at a temperature of -100 to +250°C, preferably of -78 to +150°C, particularly preferably of 0 to +40°C.

9. Process according to one of Claims 1 to 8, **characterised in that** the metathesis reaction is a ring-closing metathesis reaction of an unsaturated organic compound, and the catalyst used is a carbene complex selected from bis(tricyclohexylphosphine)benzylideneruthenium dichloride (Cl₂(Cy₃P)₂Ru=CHPh, "Grubbs" catalyst), a variant or derivative of the "Grubbs" catalyst, 2,6-diisopropylphenylimido-neophylidenemolybdenum bis(hexafluoro-tert-butoxide) (2,6-iPr₂C₆H₃N=Mo{OC(CF₃)₂Me}₂=CHCMe₂Ph, "Schrock" catalyst), a variant or derivative of the "Schrock" catalyst, or a mixture of the above-mentioned complexes, where the unsaturated organic compound is employed in an equimolar ratio or an up to 1 0,000-fold molar excess, particularly preferably in a 10-fold to 100-fold excess, very particularly preferably in a 20-fold to 30-fold excess, relative to the metathesis catalyst.

10. Process according to one of Claims 1 to 9, **characterised in that** the course of the reaction is monitored and optionally regulated chromatographically, preferably gas-chromatographically and/or by high-pressure liquid chromatography.

## Revendications

1. Procédé pour la mise en oeuvre de réactions de métathèse de composés organiques insaturés, **caractérisé en ce qu'**au moins un composé organique insaturé, qui est choisi parmi les alkènes aliphatique, aromatique ou hétéroaromatique, sous forme liquide ou dissoute, est mélangé avec au moins un catalyseur de métathèse, qui est choisi parmi les complexes carbène ou carbyne ou un mélange de ces catalyseurs sous forme liquide ou dissoute dans au moins un microréacteur, et les composés organiques insaturés réagissent pendant un temps de résidence de ≤ 1 heure dans le microréacteur ou dans un microréacteur qui est connecté à un capillaire via une sortie, et les composés organiques formés sont en option isolés du mélange de réaction, et **en ce que** le microréacteur est un réacteur continu sous la forme d'un micromélangeur statique qui présente un volume de ≤ 100 µl et de petits canaux présentant un diamètre de 10 à 1000 µm au travers desquels un écoulement est réalisé à un débit d'écoulement de 0,01 µl/min à 100 ml/min, et où les liquides et/ou les solutions sont séparés, en démarrant au niveau d'un canal d'entrée, dans chaque cas dans un plan vertical, selon deux sous-courants qui s'écoulent dans des microcanaux, les microcanaux émanants sont ensuite empilés en couches l'un au dessus de l'autre dans un élément de confluence dans un plan qui est agencé de manière à être tourné de 90° par comparaison avec le plan de dérivation.

2. Procédé selon la revendication 1, **caractérisé en ce que** le volume du microréacteur est ≤ 50 µl.

3. Procédé selon la revendication 1, **caractérisé en ce qu'**un microréacteur comportant 11 étages de mélange dont chacun présente un volume de 0,125 µl est utilisé.

4. Procédé selon la revendication 1, **caractérisé en ce que** le microréacteur est connecté à un capillaire chauffable via une sortie.

5. Procédé selon l'une des revendications 1 à 4, **caractérisé en ce que** le microréacteur est chauffable.

6. Procédé selon l'une des revendications 1 à 5, **caractérisé en ce que** le microréacteur comporte des canaux qui présentent un diamètre de 20 à 800 µm, de façon particulièrement préférable de 30 à 400 µm.

7. Procédé selon l'une des revendications 1 à 6, **caractérisé en ce que** le mélange de réaction s'écoule au travers du microréacteur à un débit d'écoulement de 1 µl/min à 1 ml/min.

8. Procédé selon l'une des revendications 1 à 7, **caractérisé en ce qu'**il est mis en oeuvre à une température de -100 à +250°C, de préférence de -78 à +150°C, de façon particulièrement préférable de 0 à +40°C.

9. Procédé selon l'une des revendications 1 à 8, **caractérisé en ce que** la réaction de métathèse est une réaction de métathèse à fermeture d'anneau d'un composé organique insaturé, et le catalyseur utilisé est un complexe carbène qui est choisi parmi dichlorure de bis(tricyclohexylphosphine)benzylidèneruthénium (Cl₂(Cy₃P)₂Ru=CHPh, catalyseur "Grubbs"), une variante ou un dérivé du catalyseur "Grubbs", 2,6-diisopropylphénylimidonéophylidènemolybdène bis(hexafluoro-tert-butoxyde) (2,6-iPr₂C₆H₃N=Mo{OC(CF₃)₂Me}₂=CHCMe₂Ph, catalyseur "Schrock"), une variante ou un dérivé du catalyseur "Schrock", ou un mélange des complexes mentionnés ci avant, où le composé organique insaturé est utilisé selon un rapport équimolaire ou un excès molaire allant jusqu'à un facteur de 10 000, de façon particulièrement préférable selon un excès allant d'un facteur de 10 à un facteur de 100, de façon très particulièrement préférable selon un excès allant d'un facteur de 20 à un facteur de 30, par rapport au catalyseur de métathèse.

10. Procédé selon l'une des revendications 1 à 9, **caractérisé en ce que** le déroulement de la réaction est surveillé et en option est régulé chromatographiquement, de préférence chromatographiquement par gaz et/ou au moyen d'une chromatographie de liquide haute pression.
